# EUROPEAN PATENT APPLICATION

(11) **EP 3 210 618 A1**
(43) Date of publication of application: **30.08.2017**
(21) Application number: 17162198.0
(22) Date of filing: 21.04.2011
(51) Int. Cl.: A61K 38/40, A61K 39/42, A61P 31/04, A61P 31/12

(54) **COMPOSITION COMPRISING LACTOFERRIN AND IMMUNOGLOBULIN FOR THE TREATMENT OF ECZEMA**

(30) Priority: 23.04.2010 AU 2010901718
(62) Divisional of application: 11771412.1
(71) Applicant: Probiotec Limited, Laverton North, Victoria 3026 (AU)
(72) Inventor: GANTER, Rudi, Victoria, 3026 (AU); AHMAD, Humera, Victoria, 3026 (AU)
(74) Representative: Lee, Nicholas John

(57) **Abstract**

A use of a composition including lactoferrin and/or immunoglobulin for minimizing the severity of one or more symptoms associated with eczema.

## Description

### Field of the invention

This application describes compositions and their use in methods for suppressing or preventing the onset of eczema, or for treating the symptoms of eczema to reduce their severity or duration. The composition provides combinations of lactoferrin and immunoglobulin.

### Background of the invention

Reference to any prior art in the specification is not, and should not be taken as, an acknowledgment or any form of suggestion that this prior art forms part of the common general knowledge in Australia or any other jurisdiction or that this prior art could reasonably be expected to be ascertained, understood and regarded as relevant by a person skilled in the art.

Eczema is a form of dermatitis or inflammation of the dermis. The term eczema is broadly applied to a range of persistent skin conditions characterised by one or more of the symptoms of redness, skin oedema (swelling), itching and dryness, crusting, flaking, blistering, cracking, oozing, or bleeding.

There are a number of different types of "common" eczema, all of which may have different causes. Atopic eczema is an allergic disease believed to have a hereditary component and often runs in families whose members also have asthma. An itchy rash is particularly noticeable on head and scalp, neck, inside of elbows, behind knees, and buttocks.

Contact dermatitis is also a type of eczema and may one of two types: allergic (resulting from a delayed reaction to an allergen), and irritant (resulting from direct reaction to an irritant). Contact eczema is curable, provided the offending substance can be avoided and its traces removed from one's environment.

Xerotic eczema is dry skin that becomes so serious it turns into eczema. It worsens in dry winter weather, and limbs and trunk are most often affected. Skin becomes very itchy and tender, with deep dry cracks appearing.

Seborrhoeic dermatitis or Seborrheic dermatitis ("cradle cap" in infants) is a condition sometimes classified as a form of eczema that is closely related to dandruff. It causes dry or greasy peeling of the scalp, eyebrows, and face, and sometimes trunk.

As eczema has a lot of leading causes, treatment can be varied. Dermatitis is often treated with corticosteroids. However prolonged use of topical corticosteroids is thought to increase the risk of possible side effects, and high-strength steroids may be absorbed into the body. Their immunosuppressive action can also lead to secondary skin infections.

Treatment for contact dermatitis may not require long term use of drugs such as corticosteroids, as treatment usually involves identification and removal of the allergen or irritant. In the meantime however, it is important to control and suppress the symptoms for the comfort of the patient.

There therefore exists a need for ongoing development of methods and compositions for suppressing the onset of eczema, or for treating the symptoms of eczema.

### Summary of the invention

In one embodiment, there is provided a use of a composition including lactoferrin and immunoglobulin for minimizing the severity of one or more symptoms associated with eczema.

In certain embodiments there is provided a use of a composition including lactoferrin and immunoglobulin in one or more of the following applications:
- reducing the duration of one or more symptoms associated with eczema in a subject;
- reducing the number of symptoms associated with eczema in a subject;
- reducing the severity or extent of one or more symptoms associated with eczema in a subject;
- reducing the complications associated with eczema in a subject;
- reducing the occurrence of one or more symptoms associated with eczema in a subject.

In other embodiments there is provided a use of a composition including lactoferrin and immunoglobulin for preventing or minimising the development of one or more symptoms associated with eczema.

In one embodiment, the composition for use in the above described embodiments includes lactoferrin and immunoglobulin.

In another embodiment, the composition for use in the above describe embodiments includes lactoferrin and immunoglobulin wherein the composition does not substantially include one or more of the following proteins: lactoperoxidase, lactoglobulin, albumin, lactalbumin.

In yet another embodiment, the composition for use in the above described embodiments consists substantially of lactoferrin and immunoglobulin.

In still another embodiment there is provided a composition including lactoferrin and immunoglobulin in synergistically effective amounts.

In a related embodiment, there is provided a method of treatment of a symptom associated with eczema including the step of administering a composition including lactoferrin and/or immunoglobulin to a subject requiring treatment.

### Detailed description of the embodiments

Reference will now be made in detail to certain embodiments of the invention. While the invention will be described in conjunction with the embodiments, it will be understood that the intention is not to limit the invention to those embodiments. On the contrary, the invention is intended to cover all alternatives, modifications, and equivalents, which may be included within the scope of the present invention as defined by the claims.

One skilled in the art will recognize many methods and materials similar or equivalent to those described herein, which could be used in the practice of the present invention. The present invention is in no way limited to the methods and materials described.

It will be understood that the invention disclosed and defined in this specification extends to all alternative combinations of two or more of the individual features mentioned or evident from the text or drawings. All of these different combinations constitute various alternative aspects of the invention.

As described herein, the inventors have surprising found that immunogobluin and/or lactoferrin -containing compositions are particularly useful for minimizing the severity of conditions associated with eczema. Key benefits of the treatment include prevent the onset of symptoms associated with eczema and/or reducing the time of symptoms in patients with eczema.

As used herein, except where the context requires otherwise, the term *"comprise"* and variations of the term, such as "*comprising*", "*comprises*" and "*comprised*", are not intended to exclude further additives, components, integers or steps.

As used herein, the phrase '*wherein the composition does not substantially include*' generally refers to a substantial, but not complete absence of a specified reagent, component or compound in the composition the subject of the phrase, specifically that the specified reagent, component or compound may be present in the composition at most in residual or trace amounts, or amounts that do not substantially influence the activity of the lactoferrin and/or immunoglobulin for minimizing one or more symptoms of eczema. In one example, the residual or trace amounts of the specified reagent, component or compound in the composition may arise from unintended contamination of the composition or from imperfect separation of lactoferrin or immunoglobulin from a precursor material used in the preparation of the composition, one example of such a material being whey.

As used herein, the phrase '*wherein the composition consists substantially of*' generally refers to a composition in which the components having the higher relative abundance or higher weight as a percentage of total weight of the composition (except for diluents, excipients, fillers and the like) are lactoferrin and/or immunoglobulin.

As used herein, the phrase '*active ingredient*' generally refers to an ingredient having activity for the treatment of a disease or condition, for example for reducing or for minimising the severity of one or more symptoms associated with eczema. Lactoferrin and immunoglobulin are active ingredients of the composition according to the invention.

As used herein, the phrase '*pharmaceutically acceptable*' generally refers to a substance or composition that is compatible chemically and/or toxicologically, with the other components included in a composition, and/or the mammal being treated therewith.

As used herein, '*synergy*' generally refers to a relationship between 2 or more components whereby when combined for use, the combined effect of the 2 or more components is quantitatively and/or qualitatively different to the sum effect arising from individual use of each component.

As used herein, the phrase '*synergistically effective amount*' generally refers to an amount of each component required to provide synergy with other components.

As used herein, '*eczema*' generally refers to a condition characterised by symptoms that are commonly observed in atopic eczema, allergic contact dermatitis, irritant contact dermatitis, xerotic eczema and seborrhoeic dermatitis. Symptoms include an itchy rash with redness and flaky skin, particularly on the head, scalp, neck, inside of elbows, behind knees, and buttocks, as well as dry and tender cracked skin, or dry or greasy peeling of the scalp, eyebrows, and face, and sometimes trunk.

As used herein, the phrase '*symptoms associated with eczema*' generally refers to itchy rash with redness and flaky skin, particularly on the head, scalp, neck, inside of elbows, behind knees, and buttocks, as well as dry and tender cracked skin, or dry or greasy peeling of the scalp, eyebrows, and face, and sometimes trunk.

As used herein, the phrase '*minimizing the severity of symptoms*' is intended to mean either a reduction of the duration of one or more symptoms associated with eczema, a reduction in the number of symptoms associated with eczema, or a reduction in the extent of one or more symptoms associated with eczema. For example, the time to resolution of eczema lesions may be reduced, or the itchiness may lessen.

As used herein, '*therapeutically effective amount*' generally refers to an amount of a composition of the present invention that (i) treats the particular disease, condition, or disorder, (ii) attenuates, ameliorates, or eliminates one or more symptoms of the particular disease, condition, or disorder, or (iii) delays the onset of one or more symptoms of the particular disease, condition, or disorder described herein.

As used herein, the words '*treat*' or '*treatment*' generally refer to therapeutic treatment wherein the object is to slow down (lessen) an undesired physiological change or disorder. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable.

As used herein, the words '*prevent*' and *'prevention'* refer to prophylactic or preventative measures for protecting or precluding an individual not having a related complication from progressing to that complication. Individuals in which prevention is required include those who have a specific condition.

As used herein, the phrase '*preventing development of one or more symptoms*' generally refers to the onset of eczema being prevented such that one or more symptoms associated with eczema do not develop at all, or do not develop to the same degree that they may have in the absence of administration of the composition including lactoferrin and immunoglobulin.

'*Lactoferrin*' (LF) also known as lactotransferrin (LTF), is a globular multifunctional protein that can be obtained from whey or related dairy products, or from recombinant technology. Lactoferrin may have a high affinity for ferrous and/or ferric ions. Lactoferrin proteolysis also produces the small peptides lactoferricin and kaliocin-1. These peptides and other lactoferrin -related peptides may, in certain embodiments, be used in addition, or in alternative to lactoferrin.

'Immunoglobulin' or 'antibody' or '*Ig*' are gamma globulin proteins that are found in milk, blood, or other bodily fluids of verterbrates that function in the immune system to bind antigen, hence identifying and neutralizing foreign objects.

Antibodies are generally a heterotetrameric glycoprotein composed of two identical light (L) chains and two identical heavy (H) chains. Each L chain is linked to a H chain by one covalent disulfide bond. The two H chains are linked to each other by one or more disulfide bonds depending on the H chain isotype. Each H and L chain also has regularly spaced intrachain disulfide bridges.

H and L chains define specific Ig domains. More particularly, each H chain has at the N-terminus, a variable domain (V_{H}) followed by three constant domains (C_{H}) for each of **the α and γ chains and four C_{H} domains for µ and ε isotypes. Each L chain has at the N-**terminus, a variable domain (V_{L}) followed by a constant domain (C_{L}) at its other end. The V_{L} is aligned with the V_{H} and the C_{L} is aligned with the first constant domain of the heavy chain (C_{H}1).

Antibodies can be assigned to different classes or isotypes. There are five classes of **immunoglobulins: IgA, IgD, IgE, IgG, and IgM, having heavy chains designated α, δ, ε, γ, and µ, respectively. The γ and α classes are further divided into subclasses on the** basis of relatively minor differences in C_{H} sequence and function, e.g., humans express the following subclasses: IgG1, IgG2, IgG3, IgG4, IgA1, and IgA2. The L chain from any vertebrate species can be assigned to one of two clearly distinct types, called kappa and lambda, based on the amino acid sequences of their constant domains.

In one aspect, there is provided a method for minimizing the severity of one or more symptoms associated with eczema.

In certain embodiments, the subject may have eczema, or other skin condition with similar or related manifestations such as dermatitis, atopic allergy and psoriasis.

In one embodiment there is provided a method for minimizing the severity of one or more symptoms associated with eczema in a subject comprising administering a therapeutically effective amount of a composition comprising lactoferrin and immunoglobulin. In a preferred embodiment, the subject has symptoms associated with eczema at the time of administration of the composition.

As exemplified herein, the method of the invention is particularly useful for reducing the duration of one or more symptoms associated with eczema in a subject.

Further, the method is effective for reducing the number of symptoms associated with eczema in a subject. In one embodiment, one or more symptoms selected from the group consisting of: itchy rash with redness and flaky skin as well as dry and tender cracked and/or bleeding skin, may be substantially obviated or ablated.

Further the severity or extent of one or more symptoms associated with eczema in a subject may be minimized or reduced.

Given that the duration of eczema symptoms are minimised with the method of the invention, or otherwise prevented, the method also finds application in reducing complications associated with eczema in a subject including skin or underlying tissue infection.

The effect of the method of the invention on symptoms of eczema in a subject can be measured by standard techniques and by following the teaching herein. Progress of treatment may be monitored by visual inspection of the areas of skin with symptoms of eczema, as well as feedback from the affected subject as to improvements in relation to itchiness, redness, pain, dryness or flakiness of skin.

According to the method, the subjects to be treated to minimize the severity of one or more symptoms associated with eczema are generally people having symptoms of eczema as described herein.

In an alterative embodiment, the subjects are asymptomatic for eczema or present with sub clinical symptoms of eczema. The subject may have a personal history of eczema or familial history of it.

Accordingly, the invention also provides a method for preventing development of one or more symptoms associated with eczema in a subject comprising administering a therapeutically effective amount of a composition comprising lactoferrin and immunoglobulin.

While the composition may be administered to any subject for preventing development of symptoms of eczema, or minimizing the severity of said symptoms, it is particularly advantageous for adults over the age of 18 who are otherwise in good health, or in people who may be more susceptible to eczema due to their age (children and infants) or the existence of a pre-existing condition which could exacerbate eczema.

In a yet a further embodiment of this aspect of the invention, the lactoferrin may be administered separately to the immunoglobulin. As such, there is provided a method for preventing development of, or minimizing the severity of, symptoms associated with eczema in a subject comprising the steps of
- administering a therapeutically effective amount of a composition of lactoferrin; and
- administering a therapeutically effective amount of a composition of immunoglobulin.

The steps of administering the lactoferrin and immunoglobulin -containing compositions may be simultaneous or sequential. Sequential administration may be immediately one after the other or up to several hours later.

The subjects to be treated according to the invention may or may not have an allergy or predisposition for allergy.

In one embodiment, the individual to be treated according to the invention does not have an allergy or otherwise is not predisposed to allergy.

In one embodiment, the composition of lactoferrin is administered first; in an alternative embodiment, the composition of immunoglobulin is administered first.

In some embodiments, it may not be necessary to administer both compositions and the prevention of the development of, or minimization of the severity of, symptoms associated with eczema in a subject can be achieved by administration of a therapeutically effective amount of a composition of lactoferrin or administration of a therapeutically effective amount of a composition of immunoglobulin.

Thus in one embodiment there is provided a method of:
- minimizing the severity of one or more symptoms associated with eczema; or
- preventing development of one or more symptoms associated with eczema;
in a subject comprising administering a therapeutically effective amount of a composition of lactoferrin to said subject. Typically the lactoferrin is provided in an orally administered formulation, for example as a capsule or liquid. The composition does not substantially include other whey proteins. In this embodiment, the composition consists substantially of lactoferrin.

Thus in one embodiment there is provided a method of:
- minimizing the severity of one or more symptoms associated with eczema; or
- preventing development of one or more symptoms associated with eczema;
in a subject comprising administering a therapeutically effective amount of a composition of immunoglobulin to said subject. Typically the immunoglobulin is provided in an orally administered formulation, for example as a capsule or liquid. The composition does not substantially include other whey proteins. In this embodiment, the composition consists substantially of immunoglobulin.

In one embodiment, the method of the invention may further include the step of administration of an active ingredient other than lactoferrin and immunoglobulin. The active ingredient may be an anti-histamine, an analgesic, an anaesthetic, an anti-inflammatory or a corticosteroid or a probiotic, such as a gram positive strain of bacteria. Other particularly useful bacteria may be Lactobacillus paracasei subsp. Paracasei, Lactobacillus acidophilus, Lactobacillus rhamnosus, Lactobacillus reuteri, Streptococcus thermophilus and Bifidobacterium lactis. The active ingredient may also be a prebiotic such as inulin, oligofructose, dextrin etc.

Due to the itchiness associated with many types of eczema, secondary skin infections may develop due to the skin barrier being broken by scratching. In the case of secondary infections the composition may include one or more anti-viral, anti-bacterial and anti-fungal agents. In one embodiment, the method includes the step of administering said further active ingredient prior to, or following administration or the composition including lactoferrin and immunoglobulin.

Accordingly, the invention also provides a method for minimizing the severity of one or more symptoms associated with eczema or for preventing development of symptoms associated with eczema in a subject, comprising administering a therapeutically effective amount of a composition comprising lactoferrin and immunoglobulin, anti-histamine, an analgesic, an anaesthetic (local), an anti-inflammatory or a corticosteroid or a probiotic.

In a further embodiment, the method includes the prior step of assessing and/or selecting an individual for the presence of, or for pre-disposition to eczema, prior to, or after treatment with the lactoferrin and immunoglobulin containing composition.

Lactoferrin and immunoglobulin may both be derived from biological fluids such as milk, blood, serum and preferably from the whey fractions of milk. Lactoferrin and immunoglobulin may also be produced recombinantly.

The immunoglobulins are preferably immunoglobulin G (IgG) selected from one or more subclasses of IgG1, 2, 3 and 4, and immunoglobulin A (IgA) of either of both of subclass IgA1 and 2. Immunoglobulin M (IgM) may also be included in some embodiments, together or without IgE and/or IgD. In one embodiment, the composition may be enriched for one or more immunoglobulin classes or subclasses.

In one embodiment, the lactoferrin and/or immunoglobulin are provided for use in the form of a whey or related whey product as mentioned below.

Where the lactoferrin and/or immunoglobulin are provided for use in the form of a whey or related whey product, the whey or whey product may have had a pharmaceutically acceptable carrier added to it.

"Whey" is generally the serum or watery fraction of milk that forms along with curd formation when milk coagulates. Whey may be formed from any mammalian milk, preferably cow, goat or sheep milk, more preferably cow milk. Whey is typically formed in cheese production when curd forms. Whey is rich in lactoferrin, immunoglobulins, alpha lactalbumin, beta lactoglobulin, glycomacropeptide, serum albumin, lactoperoxidase, and other minerals and vitamins.

"Sweet whey" may be derived from the manufacture of cheeses such as Cheddar, Mozzarella and Swiss that has been pasteurized and to which no preservatives have been added. Sweet whey powder generally contains all the constituents of fresh whey, except water in the same relative proportion. Typically, sweet whey powder has about 11-14.5% protein, about 63 to 75% lactose, about 1 to 1.5% fat, about 7.2 to 8.8% ash and about 3.5 to 5% moisture.

"Acid whey" may be derived from the manufacture of cheeses such as cottage, cream cheese and ricotta that has been pasteurized and to which no preservatives have been added. Acid whey powder contains all the constituents of original acid whey, except water, in the same relative proportion. Typically, acid whey powder has about 11-13.5% protein, about 61 to 70% lactose, about 0.5 to 1.5 % fat, about 9.8 to 12.3 % ash and about 3.5 to 5% moisture. Acid whey may be neutralised before use in the invention described herein.

"Reduced lactose whey" may be obtained by the selective removal or hydrolysis of lactose from whey. The lactose content of the dry product may not exceed 60%. Reduction of lactose may be accomplished by physical separation techniques such as precipitation or filtration or enzymatic hydrolysis of lactose to glucose and galactose. The acidity of reduced lactose whey may be adjusted by the addition of safe and suitable ingredients. Typically, reduced lactose whey powder has about 18-24% protein, about 52 to 58% lactose, about 1 to 4 % fat, about 11 to 22 % ash and about 3 to 4% moisture.

"Demineralised whey" (also called 'reduced -minerals whey') is obtained by removing a portion of the minerals from pasteurized whey. Typical levels of demineralization are 25%, 40%, 50%, 70% and 90%. The dry product may not exceed 7% ash. Demineralised whey may be produced by separation techniques such as ion exchange, diafiltration or electrodialysis. The acidity of demineralised whey may be adjusted by the addition of safe and suitable ingredients.

"Whey protein" is generally the protein that is found in whey. It may be provided in the form of a solid, liquid or concentrate.

A "whey protein extract" includes whey proteins. The whey protein extract may be a raw extract or eluate that has undergone minimal further processing/purification after separation from casein, some proteins and dairy fat. In some embodiments, the whey protein extract consists essentially of whey protein. In these embodiments, the composition substantially lacks proteins that are not found in whey, although the composition may include other non protein components such as carbohydrate and fat. Examples include whey protein concentrates (WPC) and whey protein isolates (WPI).

WPCs, are concentrates of whey having specified amounts of protein. Generally, WPC34 specifies a concentrate having not less than 34% protein, WPC50 specifies a concentrate having not less than 50% protein, WPC60 specifies a concentrate having not less than 60% protein, WPC75 specifies a concentrate having not less than 75% protein and WPC80 specifies a concentrate having not less than 80% protein. These concentrates may be formed by ultrafiltration of pasteurised whey, recovery of the retentate, followed by concentration and spray drying of the retentate to form WPC34 and WPC50 powder; or diafiltration of the retentate, followed by concentration and spray drying to form WPC50, WPC60, WPC75 or WPC80.

WPI is obtained by removing sufficient non protein constituents from whey so that the finished dry product contains not less than 88% protein. WPI is produced by membrane separation processes or ion exchange. In one example, pasteurized fluid whey is subjected to microfiltration resulting in removal of lipids, diafiltration is then applied to form a permeate and whey protein isolate and the latter is then concentrated and spray dried to form WPI powder. In another example, pasteurized fluid whey is subjected to ion exchange protein separation leading to deproteinated whey and the adsorbed whey protein is then desorbed, subjected to ultrafiltration or further ion exchange to remove minerals. The whey protein isolate so formed is then subjected to concentration and spray drying to form WPI powder.

In one embodiment, the whey protein extract may be enriched for whey protein. For example, whey protein may be added to a whey protein extract to increase the relative abundance of whey protein in the extract. In other embodiments, non whey protein, or other non protein components may be removed from a whey protein extract, thereby increasing the relative abundance of whey protein in the composition. In one embodiment, the whey protein extract may be an essentially pure whey protein extract enriched for lactoferrin or immunoglobulin by separation from the whey components of alpha lactalbumin, beta lactoglobulin, glycomacropeptide, serum albumin, and lactoperoxidase.

Whey protein extracts may be further enriched for lactoferrin or immunoglobulins, or both by addition of an essentially pure whey protein extract enriched for lactoferrin or immunoglobulin to a whey protein extract that has not undergone enrichment for one or both of lactoferrin, immunoglobulin. Whey protein extracts may be alternatively enriched for lactoferrin or immunoglobulins by the addition of one or both of recombinant lactoferrin an immunoglobulin.

Essentially pure extracts of lactoferrin and immunoglobulin derived from a whey protein extract may each be optionally milled to the desired particle size.

The protein components of the whey protein extract may also be partially or extensively hydrolysed. A "partially hydrolysed whey protein extract" generally contains oligopeptides that have a molecular weight of generally less than 5000d. An "extensively hydrolysed whey protein extract" generally contains peptides that have a molecular weight of less than 3000d.

In one embodiment, the amount of protein in the whey protein extract is at least about 10% w/w on a dry weight basis.

In one embodiment, the amount of protein is about 10% to less than about 90% w/w.

In one embodiment, the amount of protein is about 11 % to about 25% w/w.

In one embodiment, the amount of protein is about 11 % to about 18% w/w.

In one embodiment, the amount of protein is about 18% to about 25%w/w.

In one embodiment, the amount of protein is about 34% to about 80% w/w.

In one embodiment, the amount of protein is about 50% to about 75% w/w.

In one embodiment, the amount of protein is about 50% to about 60% w/w.

In one embodiment, the amount of protein is about 60% to about 75% w/w.

In one embodiment, the amount of protein is about 90% to about 95% w/w.

In one embodiment, the whey protein extract includes at least one of carbohydrate (such as lactose) and fat.

In one embodiment, the amount of lactose in the whey protein extract is at least about 1 % w/w on a dry weight basis.

In one embodiment, the amount of lactose is about 1% to less than about 80%.

In one embodiment, the amount of lactose is about 63% to about 75% w/w.

In one embodiment, the amount of lactose is about 61 % to about 70% w/w.

In one embodiment, the amount of lactose is about 52% to about 58% w/w.

In one embodiment, the amount of lactose is about 70% to about 80% w/w.

In one embodiment, the amount of lactose is about 48% to about 52% w/w.

In one embodiment, the amount of lactose is about 33% to about 37% w/w.

In one embodiment, the amount of lactose is about 25% to about 30% w/w.

In one embodiment, the amount of lactose is about 10% to about 15% w/w.

In one embodiment, the amount of lactose is about 4% to about 8% w/w.

In one embodiment, the amount of lactose is about 0.5% to about 1.0% w/w.

In one embodiment, amount of fat in the whey protein -containing composition is at least about 0.5% w/w on a dry weight basis.

In one embodiment, the amount of fat is about 0.5% to less than about 10% w/w.

In one embodiment, the amount of fat is about 1% to about 5% w/w.

In one embodiment, the amount of fat is about 5% to about 7% w/w.

In one embodiment, the whey protein -containing composition includes about 80 to 82% protein, about 4 to 8% lactose and about 4 to 8% fat w/w.

As an alternative to whey, lactoferrin to be used in the method of the invention may be recombinant lactoferrin or may be isolated from any biological fluid including milk. As detailed above, following pasteurisation of milk (the milk may be unpasteurised), cheese is manufactured from the milk, resulting in the removal of casein, some protein and dairy fat, leaving a liquid whey stream containing (among other things) lactoferrin. The lactoferrin in the composition of the invention may therefore be provided in the form of a whey protein extract or as recombinantly produced lactoferrin. When it is a whey protein extract, the extract may be a raw extract or eluate ie minimal further processing/purification after separation from casein, protein and dairy fat.

Alternatively, the method of the invention may use a composition that consists substantially of lactoferrin and immunoglobulin as the only active ingredients, in which case the lactoferrin has been subjected to additional purification steps such that the lactoferrin is provided in the form of an essentially pure fraction or eluate of whey protein extract, optionally milled to the desired particle size. Compositions that consist substantially of lactoferrin and immunoglobulin may comprise recombinantly produced lactoferrin.

The whey protein extract may alternatively be enriched for lactoferrin, by inclusion in the composition of a whey protein extract together with lactoferrin provided in the form of an essentially pure fraction separated from other whey proteins, or by addition of recombinantly produced lactoferrin.

In yet a further embodiment, the whey product extract is a fraction from which lactoferrin has been removed (purified out) but the extract is supplemented with one or both of an essentially pure lactoferrin extract separated from immunoglobulins, alpha lactalbumin, beta lactoglobulin, glycomacropeptide, serum albumin, and lactoperoxidase.

As detailed above, the whey protein extract may be a raw fraction or extract, an essentially pure lactoferrin extract separated from immunoglobulins, alpha lactalbumin, beta lactoglobulin, glycomacropeptide, serum albumin, and lactoperoxidase, or a combination of the two to provide a lactoferrin enriched extract. The lactoferrin enriched extract may also be produced by addition of recombinantly produced lactoferrin to a raw fraction or extract.

Immunoglobulins may be provided as recombinant immunoglobulin, immunoglobulin isolated from blood or other body fluid, or as an extract obtained during milk processing and cheese production processes. Immunoglobulins to be used in the composition of the invention may be isolated from pasteurised milk. The milk may be human milk, bovine milk, or milk from another mammal. As detailed above, following pasteurisation of milk, cheese is manufactured from the milk, resulting in the removal of casein, some protein and dairy fat, leaving a liquid whey stream containing (among other things) immunoglobulins, particularly IgG and IgA. The immunoglobulins in the composition of the invention may be provided in the form of a whey protein extract or as recombinant immunoglobulin. When it is a whey protein extract, the extract may be a raw extract or eluate ie minimal further processing/purification after separation from casein, protein and dairy fat.

Alternatively, when the composition of the invention consists essentially of lactoferrin and immunoglobulin as the only active ingredients, the immunoglobulin is subjected to additional purification steps such that the immunoglobulin is provided in the form of an essentially pure fraction or eluate of whey protein extract, optionally milled to the desired particle size. Compositions that consist essentially of lactoferrin and immunoglobulin may alternatively comprise recombinantly produced immunoglobulin.

The whey protein extract may alternatively be enriched for immunoglobulins, by inclusion in the composition of a whey protein extract together with immunoglobulins provided in the form of an essentially pure fraction separated from other whey proteins or by addition of recombinantly produced immunoglobulin.

In yet a further embodiment, the whey protein extract is a fraction from which immunoglobulin has been removed (purified out) but the extract is supplemented with one or both of an essentially pure immunoglobulin extract separated from lactoferrin, alpha lactalbumin, beta lactoglobulin, glycomacropeptide, serium albumin, and lactoperoxidase or recombinant immunoglobulin.

As detailed above, the whey protein extract may be a raw fraction or extract, an essentially pure immunoglobulin extract separated from lactoferrin, alpha lactalbumin, beta lactoglobulin, glycomacropeptide, serum albumin, and lactoperoxidase, or a combination of the two to provide an immunoglobulin enriched extract. The immunoglobulin enriched extract may also be produced by addition of recombinantly produced immunoglobulin to a raw fraction or extract. In some embodiments, the whey protein extract has already been purified of immunoglobulin.

As whey protein extracts can contain both lactoferrin and immunoglobulin, the lactoferrin and immunoglobulin in the composition of the invention may both be provided in the form of a whey protein extract.

The whey protein extract may be one or more of:
- a raw fraction or extract;
- an essentially pure immunoglobulin extract separated from lactoferrin, alpha lactalbumin, beta lactoglobulin, glycomacropeptide, serum albumin, and lactoperoxidase together with an essentially pure lactoferrin extract separated from immunoglobulin, alpha lactalbumin, beta lactoglobulin, glycomacropeptide, serum albumin, and lactoperoxidase;
- a combination of a raw fraction or extract with one or both of the essentially pure extracts to provide an immunoglobulin and/or lactoferrin enriched extract;
- a combination of a raw fraction or extract with one or both of recombinant lactoferrin and immunoglobulin.

Alternatively, where one or both of lactoferrin and immunoglobulin have been purified or isolated out of the whey protein extract, lactoferrin and immunoglobulin may be provided to the extract in the form of an essentially pure immunoglobulin extract separated from lactoferrin, alpha lactalbumin, beta lactoglobulin, glycomacropeptide, serum albumin, and lactoperoxidase and/or an essentially pure lactoferrin extract separated from immunoglobulin, alpha lactalbumin, beta lactoglobulin, glycomacropeptide, serum albumin, and lactoperoxidase.

As described herein, the invention relates to a therapeutic composition of one or both of lactoferrin and immunoglobulin to prevent development of, or minimizing the severity of, one or more symptoms associated with eczema. Preferably, the lactoferrin and immunoglobulin are present in the composition in synergistically effective amounts. In one embodiment, the lactoferrin and immunoglobulin may be present in the composition in amounts of a 1:1 ratio, i.e. g/g, or mass:mass ratio.

In a preferred embodiment of this aspect of the invention, a capsule, caplet, tablet or like includes lactoferrin in an amount from about 180mg to 250mg, and immunoglobulin in an amount from about 90 to 100mg. In a preferred embodiment, a capsule, caplet, tablet or like includes 220mg of lactoferrin, 104mg of immunoglobulin, and 5.4mg of magnesium stearate.

In another embodiment of this aspect of the invention, a capsule, enteric or film coated caplet, enteric or film coated tablet, powder sachet or like includes lactoferrin in an amount from about 50mg to 2,000mg, and immunoglobulin in an amount from about 50 to 2,000mg. In a preferred embodiment, a capsule, caplet, tablet or like includes 250mg of lactoferrin and 250mg of immunoglobulin.

The composition of the invention may also be formulated as a liquid. A liquid formulation may contain 2-3% w/w of lactoferrin and 1.0-1.3% w/w of immunoglobulin, more preferably 2.5% w/w lactoferrin and 1.2% w/w immunoglobulin.

Further, a liquid formulation may contain 1.0-20% w/w of lactoferrin and 1.0-20% w/w of immunoglobulin, more preferably 5% w/w lactoferrin and 5% w/w immunoglobulin.

It is especially advantageous to formulate the compositions of the present invention in unit dosage form for ease of administration and uniformity of dosage. The specifications for the dosage unit forms of the present invention may be determined by a person skilled in the art together with the information provided below, depending on, for example (a) the characteristics of the lactoferrin and immunoglobulin and the particular therapeutic effect to be achieved, and (b) the limitations inherent in the art of compounding such active ingredients for the particular treatment.

Generally when administered for therapy of eczema, the composition may be administered as two capsules, caplets, tablets or like (as described above), or 10 - 20 mls of liquid formulation, (as described above) every 4 hours.

Where prevention is intended, the composition is administered in as 2 capsules, caplets, tablets or like (as described above), or 10 - 20 mls of liquid formulation, (as described above) daily.

Typically the dosing will be for 1 to 12 weeks where treatment is required.

Preferably about 50 to 2000mg, more preferably about 50 to 600mg of lactoferrin is administered per day. Preferably about 1 to 2000mg, more preferably about 50 to 600mg of immunoglobulin is administered per day.

It may be beneficial, for symptomatic patients in particular, for the composition of the invention to include a further active pharmaceutical or principle. These may be incorporated into the therapeutic composition, depending on the anticipated route of administration and the stage of the infection or related complications. For example, the therapeutic composition may further include an anti-histamine or an analgesic or a corticosteroid. In another embodiment, the further active pharmaceutical or principle is a probiotic. In the case of co-infections or secondary infections, the therapeutic composition may include one or more anti-viral, anti-bacterial, anti-fungal and anti-protozoan agents.

The composition may further comprises other pharmaceutically acceptable diluents, carriers, excipients or like compounds. Acceptable diluents, carriers, excipients, and stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as plasma albumin and gelatin; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The composition may further comprise preservatives, anti-microbial agents, colorings and flavorings.

The composition may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Caplets, capsules, tablets and the like may be enteric or film coated.

In general the formulations are prepared by uniformly and intimately bringing into association the active ingredient with liquid carriers or finely divided solid carriers or both, and if necessary, shaping the product. Formulation may be conducted by mixing at ambient temperature at the appropriate pH, and at the desired degree of purity, with physiologically acceptable carriers, i.e., carriers that are non-toxic to recipients at the dosages and concentrations employed. The pH of the formulation depends mainly on the particular use and the concentration of compound, but may range from about 3 to about 9. Formulation in an acetate buffer at pH 5 is a suitable embodiment. The inhibitory compound for use herein is preferably sterile. The compound ordinarily will be stored as a solid composition, although lyophilized formulations or aqueous solutions are acceptable. Thus in certain embodiments there is provided a use of a composition as described above in the manufacture of a composition for treating eczema in an individual.

The composition may be packaged in a variety of ways depending upon the method used for administering the drug. Generally, a kit or article for distribution includes a container having deposited therein the pharmaceutical formulation in an appropriate form. Suitable containers are well-known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

It is especially advantageous to formulate the compositions of the present invention in unit dosage form for ease of administration and uniformity of dosage. The specifications for the dosage unit forms of the present invention may be determined by a person skilled in the art depending on, for example (a) the characteristics of the lactoferrin and immunoglobulin and the particular therapeutic effect to be achieved, and (b) the limitations inherent in the art of compounding such active ingredients for the particular treatment.

In other embodiments there is provided a kit for use in one of the above described embodiments, the kit including:
- a container holding a therapeutic composition;
- a label or package insert with instructions for use.

In certain embodiments the kit may contain one or more further active principles or ingredients for treatment of an infection or for preventing an infection- related complication described above.

The kit or "article of manufacture" may comprise a container and a label or package insert on or associated with the container. Suitable containers include, for example, bottles, vials, syringes, blister pack, etc. The containers may be formed from a variety of materials such as glass or plastic. The container holds a therapeutic composition which is effective for treating the condition and may have a sterile access port (for example the container may be an intravenous solution bag or a vial having a stopper pierceable by a hypodermic injection needle). The label or package insert indicates that the therapeutic composition is used for treating the condition of choice. In one embodiment, the label or package insert includes instructions for use and indicates that the therapeutic composition can be used to treat an infection or to prevent a complication stemming from infection.

The kit may comprise (a) a therapeutic composition; and (b) a second container with a second active principle or ingredient contained therein. The kit in this embodiment of the invention may further comprise a package insert indicating that the other active principle can be used to treat a disorder or prevent a complication stemming from infection. Alternatively, or additionally, the kit may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Any one of the following processes or modifications may be applied for preparation of a composition for use in the method of the invention:
- enrichment, concentration or fractionation of whey protein, for example using membrane filtration technology discussed further herein;
- enzymatic hydrolysis to partially or extensively hydrolyse protein contained in the extract;
- mineral or ash removal, for example using electrodialysis technology further discussed herein;
- removal of non protein components using chromatography or crystallisation technologies further described herein;
- addition of immunoregulatory molecules;
- further drying
- sizing of granules.

Membrane technologies including reverse osmosis, nanofiltration, ultrafiltration and microfiltration may involve a pressure -driven separation using a semi -permeable membrane whereby a combination of pumps and valves creates a pressure gradient across the membrane which drives the smaller molecules in the whey across the membrane, thereby concentrating the larger molecules and particles that cannot cross the membrane. Selective separation or concentration can be achieved by using membranes with different pore sizes or molecular weigh cut-off. Examples of membranes include those used in reverse osmosis (RO), nanofiltration (NF), ultrafiltration (UF) and microfiltration (MF). UF membranes have the smallest pores and allow only water to cross the membrane while all other components of the whey are retained. A common use of RO membranes is water desalination. These membranes are generally rated according to their ability to reject salt. Like vacuum concentration, RO systems do not change the ratios of the solid components of the whey but rather concentrate the solid components by removing only water. The extent of whey concentration by RO is limited by the increase in viscosity and osmotic pressure of the whey as water is removed.

Nanofiltration membranes are sometimes referred to as "loose" RO membranes. NF membranes allow some monovalent ions to cross the membrane along with the water resulting in a partial "desalting" of the whey. Because only minerals with a single charge are removed, NF membranes only slightly reduce the mineral content of whey. NF membranes may be used to reduce the sodium chloride content of some types of whey.

Ultrafiltration membranes have larger pores than RO or NF membranes. UF membranes permeate lactose and ash while retaining the proteins in whey thereby making UF membranes the standard tool for production of WPCs. The greater the amount of lactose and ash removed, the higher the protein content of the WPC. Because the viscosity of the whey increases as protein concentrate increases, addition of water to the retentate to wash out additional amounts of lactose and minerals, in a process known as diafiltration, is necessary when producing WPCs with more than 50% protein.

Microfiltration membranes have the largest pores of the membrane separation processes. Smaller soluble proteins, peptides, lactose, minerals, non-protein nitrogen components, and water readily permeate MF membranes. Fat globules are retained by MF membranes therefore these membranes can be used to remove the small amounts of fat that are not recovered by centrifugation. Trace amounts of fat must be removed to produce WPIs.

Electrodialysis also uses semi-permeable membranes, however, an electrical current replaces pressure as the driving force for separating whey components. Electrodialysis membranes allow only minerals to permeate while retaining lactose and proteins. An electrical current draws the charged mineral ions through the membranes and into a brine stream. Lactose is not affected by the current and proteins cannot cross the membranes. Electrodialysis does not denature whey proteins while removing up to 75% of the minerals in whey.

Ion exchange is a type of chromatography. For example, when producing demineralized whey, whey passes through a column containing absorbent beads that bind the ions (charged minerals) in the whey. The remainder of the whey components such as protein and lactose pass through the column unhindered. The resulting whey therefore will have reduced amounts of minerals as compared to untreated whey. Ion exchange does not denature proteins and can remove up to 98% of the minerals in whey.

Chromatography processes use charged resins to separate proteins in whey from other components. The proteins bind to oppositely charged resin while components like lactose do not bind and therefore pass directly through the system. After the whey has passed through the column or tank containing the resin, a buffer is sent through the system to release the bound proteins. The proteins can be purified further by UF and then spray dried.

Chromatography can also be used to separate specific proteins from other proteins in whey. Lactoferrin and lactoperoxidase are positively charged at a pH typical for sweet whey. The major proteins of whey, Alpha-lactalbumin, Beta-lactoglobulin and bovine serum albumin are negatively charged at the same pH. When whey passes through a tank containing negatively charged resin, the positively charged lactoferrin and lactoperoxidase bind to the resin while the other proteins and whey constituents pass through the column. An alkaline solution is then sent through the column to release the bound proteins from the resin. The recovered proteins can then be washed and spray dried.

Crystallization is used to produce either lactose or non-hygroscopic whey/permeate powder. Whey or permeate is concentrated to at least 50% total solids by evaporation where lactose is supersaturated such that the lactose will readily crystallize as the concentrated whey/permeate is cooled. After the whey/permeate has cooled sufficiently, the lactose crystals can be removed for further processing into high quality lactose, or the whey/permeate solution with crystallized lactose can be dried to produce non-hygroscopic whey/permeate powder.

Beta-galactosidase, an enzyme, can be added to whey to hydrolyze the disaccharide lactose into its component monosaccharides, glucose and galactose. Time and temperature are used to control the degree of lactose hydrolysis.

Proteases are enzymes added to whey to hydrolyze the proteins. The type of protease added, time and temperature are used to control the type and degree of protein hydrolysis.

In principle, protein modification by hydrolysis is the opposite of polymerization. Proteases are the most common group of enzymes used to cleave the peptide bonds of a protein molecule, resulting in smaller peptides and polypeptides. The degree of hydrolysis, i.e., the degree to which the whey proteins are hydrolyzed, will affect the functional properties of the hydrolysates as food ingredients.

Whey proteins can be denatured by heat to alter their functional properties. A combination of time and temperature is used to control the amount of whey protein denaturation. Controlled denaturation often is done during the preheating treatment. The amount of undenatured whey protein can be measured by the whey protein nitrogen index.

### Examples

### Example 1. Tablet formulation of the composition

An example of a formulation is as follows:

| **Ingredient** | **Range of ingredient per Tablet** | **Preferred amount of ingredient per tablet** | **Preferred amounts (w/w%)** |
|---|---|---|---|
| Lactoferrin | 50-250mg | 250mg | 20 - 80% |
| Immunoglobulin | 50-250mg | 250mg | 20 - 80% |

### Example 2. Liquid formulation of the composition

| Ingredient | Range of ingredient % w/w | Preferred amount of ingredient % w/w |
|---|---|---|
| Lactoferrin | 2-3 | 2.4 |
| Immunoglobulin | 2.3 | 2.4 |
| Sodium benzoate | 0.1-0.2 | 0.1 |
| Nipasept (preservative) | 0.05-0.1 | 0.075 |
| Povidone | 0.250-1 | 0.500 |
| Sodium carboxymethylcellulose | 0.025-0.075 | 0.050 |
| Anhydrous citric acid | 0.01-0.03 | 0.0209 |
| Monobasic potassium phosphate | Up to 0.0002 | 0.0001 |
| Glycerol | 10-15 | 20.00 |
| Strawberry flavour | Up to 0.1 | 0.050 |
| Vanilla flavour | Up to 0.1 | 0.050 |
| Amaranth | Up to 0.002 | 0.001 |
| Water | To 100 | |

### Example 3 Gel formulation

A gel formulation is as follows:

| **Ingredient** | **Range of ingredient % w/w** | **Preferred amount of ingredient % w/w** |
|---|---|---|
| Lactoferrin | 0.1-25 | 10.5 |
| Immunoglobulin | 0.1-25 | 10.5 |
| Cetomacrogol 1000 BP | 1-3 | 1.5 |
| Cetostearyl Alcohol BP | 5-10 | 6.0 |
| Paraffin - White soft BP | 2-7 | 5.0 |
| Paraffin - Liquid BP | 2-7 | 5.0 |
| Glycerol | 5-15 | 10.0 |
| Germaben II | Up to 1 | 0.60 |
| Colostrum powder | 2-7 | 5.00 |
| Water | To 100 | |

An alternative gel formulation is as follows:

| **Ingredient** | **Range of ingredient % w/w** | **Preferred amount of ingredient % w/w** |
|---|---|---|
| Lactoferrin | 5-15 | 5.5 |
| Immunoglobulin | 5-15 | 5.5 |
| Cetomacrogol 1000 BP | 1-3 | 1.5 |
| Cetostearyl Alcohol BP | 5-10 | 6.0 |
| Paraffin - White soft BP | 2-7 | 5.0 |
| Paraffin - Liquid BP | 2-7 | 5.0 |
| Glycerol | 5-15 | 12.0 |
| Germaben II | Up to 1 | 0.60 |
| Colostrum powder | 2-7 | 5.00 |
| Water | To 100 | |

### Example 4 Clinical trial demonstrating minimisation of severity of symptoms associated with eczema.

In a clinical trial of a lactoferrin / immunoglobulin preparation directed towards an unrelated indication, an outcome with respect to amelioration of skin problems was observed. In a cohort of 104, with 51 taking a placebo, 12 participants within the test group presented with skin problems.
N=9 minor skin irritations / eczema on arms
N=2 skin irritations / eczema on one leg
N=1 severe eczema on legs for 12 yrs

### 1 Method

Randomized, double-blind (participants and investigators), placebo-controlled trial.

Participants were allocated one of the following treatments before breakfast for 90 days:
i) Test 600 mg/d [2x 300 mg] lactoferrin / immunoglobulin; or
ii) Placebo 600 mg/d [2x CaHP].

### 2 Study Particulars

### i) Inclusion criteria

**Self report ≥ 1 colds per month;**
M or F, 18 yrs or over;
Good general health;

Participants of childbearing age who agree to continue using birth control measures for the duration of the study.

### ii) Exclusion criteria

Use of vitamins, herbal preparations and probiotics or any other medications for one week prior to beginning treatment and for the duration of the study;
Use of cold and flu or Sudafed while on the study;
History of alcohol or substance abuse;
Female participants who are lactating, pregnant or planning to become pregnant;

History of serious or unstable cardiac, renal, hypertensive, pulmonary, endocrine, neurologic or neuropsychiatric disorders.
*N = 104 evaluable participants*

### iii) Follow-up period

Visit 1 - baseline screening;
Visit 2 - 45 days;
Visit 3 - 90 days.

### 3 Results

The results showed beneficial outcomes for all individuals with eczema, specifically amelioration of skin problems was reported.

### Example 5 Process for production of immunoglobulin for use in composition

When cheese is manufactured from milk (pasteurised or unpasteurised), this results in the removal of casein, some protein and dairy fat, leaving a liquid whey stream from which lactoferrin and immunoglobulins may be isolated.

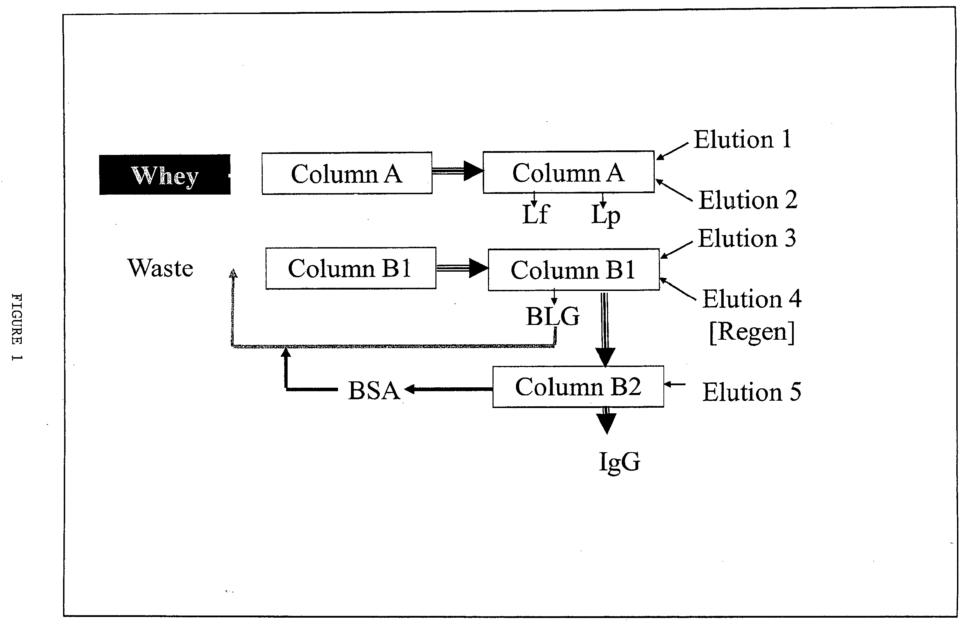

Flow chart in Figure 1 illustrates the overall process.
1. The liquid whey stream is first clarified via mechanical clarifier to remove any cheese fines solids
2. Column A is loaded with the clarified liquid whey stream then rinsed with water.
3. Elution with phosphate and chloride at pH 6 to 7 on Column A produces an eluant containing lactoperoxidase.
4. Elution with alkali at ambient temperature Column A produces an eluant containing lactoferrin. In turn, the pH of the lactoferrin eluate is adjusted to 7.
5. the pH of the run through from Column A (ie whey minus lactoperoxidase and lactoferrin) is adjusted to pH 4 to 5 with acid and loaded on to column B1.
6. The run through from step 5 is adjusted to a pH of 5 to 6 with alkali and reapplied to Column B1 to elute β-lactoglobulin.
7. Following a rinse of the column with water, alkali is applied to Column B1 to elute liquid immunoglobulins & Bovine Serum Albumin (BSA).
8. The pH of the liquid immunoglobulins and BSA fraction is adjusted to pH 4.5 to 5.0 with acid and loaded on to column B2.
9. BSA is trapped on Column B2 and the run through from Column B2 contains purified whey protein immunoglobulins removed for micro filtration step

Whey protein immunoglobulins are then subjected to ultrafiltration step to produce a concentrated 2% solids solution of purified whey protein immunoglobulins that can be freeze dried or spray dried (20% solids solution of purified whey protein immunoglobulins stored and transported into tanks at 0-4°C).

### Example 6 Immunoglobulin

Immunoglobulin may have the following composition:

| **Component** | **Amount** | **Method of determining amount** |
|---|---|---|
| Protein (% as is) | ≥ 90% | AS2300.1.2.1-(1991) |
| Immunoglobulins (%, IgG) | ≥ 30% | HPLC |
| Immunoglobulins (%, IgA) | ≥ 5% | ELISA |
| Fat | ≤ 1% | AS2300.1.3 (1998) |
| Moisture | ≤ 7% | AS2300.1.1 (1998) |
| Ash | ≤ 1.5% | AS2300.1.5 (1998) |

### Example 7 Lactoferrin

Lactoferrin may have the following composition:

| **Component** | **Amount** |
|---|---|
| Fat | < 1 % |
| Moisture | < 6 % |
| Ash | < 1.5 % |
| pH (1 % Solution) | 6-7 |
| **Heavy metals:** | **≤ 10 ppm** |
| Protein (%, as is) | > 90 % |
| Lactoferrin (% of Protein, HPLC) | > 85 % |

### Embodiments of the present invention are described in the following numbered paragraphs.

1. A use of a composition including lactoferrin and/or immunoglobulin for minimizing the severity of one or more symptoms associated with eczema.
2. A use of a composition including lactoferrin and/or immunoglobulin for preventing or minimising the development of one or more symptoms associated with eczema.
3. The use according to any one of the preceding paragraphs wherein the composition includes lactoferrin and immunoglobulin.
4. The use according to any one of the preceding paragraphs wherein the composition does not substantially include one or more of the following proteins: lactoperoxidase, lactoglobulin, albumin.
5. The use according to any one of the preceding paragraphs wherein the composition consists substantially of lactoferrin and/or immunoglobulin.
6. A use of a composition including lactoferrin and immunoglobulin for minimising the severity of one or more symptoms associated with eczema, wherein the lactoferrin and immunoglobulin are present in a mass ratio of 1:1.
7. A use according to paragraphs 6, wherein the composition includes 250mg of lactoferrin and 250mg of immunoglobulin.
8. A use according to any one of the preceding paragraphs, wherein the composition is provided in the form of a capsule, caplet, tablet, gel or syrup.

## Claims

1. A composition consisting substantially of lactoferrin and immunoglobulin for use in (a) minimizing the severity of, or (b) preventing or minimising the development of, one or more symptoms associated with eczema, wherein the lactoferrin and immunoglobulin in the composition are obtainable from a whey fraction of milk, wherein the lactoferrin and immunoglobulin are present in a mass ratio of 1:1, and wherein the composition is orally administered.

2. The composition for use according to claim 1, wherein the composition includes 250mg of lactoferrin and 250mg of immunoglobulin.

3. The composition for use according to any one of the preceding claims, wherein the composition is provided in the form of a capsule, caplet, tablet, gel or syrup.
